# EUROPEAN PATENT APPLICATION

(11) **EP 1 719 781 A2**
(43) Date of publication of application: **08.11.2006**
(21) Application number: 06017014.9
(22) Date of filing: 08.08.1997
(51) Int. Cl.: C08B 37/00

(54) **Method of introducing an amine-reactive functionality into a dextran**

(62) Divisional of application: 97937122.6
(71) Applicant: Dade Behring Marburg GmbH, 35041 Marburg (DE)
(72) Inventor: Mehta, Harshvardhan, Santa Clara California 95050 (US); Singh, Rajendra, San Jose California 95135 (US)
(74) Representative: Schweitzer, Klaus

(57) **Abstract**

The present invention discloses a method for introducing an amine-reactive functionality into a dextran. The method comprises (a) reacting the dextran with an alkylating agent having a functionality that reacts with an hydroxyl group of the dextran thereby forming an alkylated dextran wherein the alkylating agent has an olefin group and (b) treating the alkylated dextran to convert the olefin group to an amine-reactive functionality.

## Description

### Background of the Invention

### Field of the Invention

In the fields of medicine and clinical chemistry, many studies and determinations of physiologically reactive species such as cells, proteins, enzymes, cofactors, nucleic acids, substrates, antigens, antibodies, and so forth are carried out using conjugates involving specific binding pair members or labels or the like. Various assay techniques that involve the binding of specific binding pair members are known. These assay techniques generally also involve a label used in the detection part of the assay.

Polysaccharides, particularly dextran, have been conjugated to specific binding pair members to increase the stability of the specific binding pair member. Conjugation of these members to polysaccharides also increases the bulkiness of these molecules, which can enhance their effectiveness in assays involving specific binding pair members by interfering with binding to complementary specific binding pair members. Additionally, these conjugates, when present on a surface, permit specific binding of a complementary specific binding pair member to the surface with greatly reduced non-specific binding.

Aminodextran or carboxymethyldextran have usually been utilized for forming conjugates to specific binding pair members. Coupling the dextran to a protein, for example, can then be carried out through formation of an amide.

However, aminodextrans and carboxymethyldextrans have a charge that often must be neutralized to control non-specific binding. Such neutralization is difficult to do without derivitizing the conjugate biomolecule at the same time.

An alternative method of conjugation is to first partially oxidize the polysaccharide with periodate to introduce aldehyde groups. Coupling to amine containing ligands and receptors can then be carried out by reductive amination. Although dextrans that are partially oxidized are not charged, the oxidation is difficult to precisely control, and the products have substantially reduced stability toward hydrolysis. A procedure for introducing aldehyde groups onto polysaccharides that does not compromise stability, is more readily carried out, and that permits ready conjugation is therefore needed.

### Description of the Related Art

Lauritzen, et al., discuss dot immunobinding and immunoblotting of picogram and nanogram quantities of small peptides on activated nitrocellulose in Journal of Immunological Methods (1990) 131:257-267.
Effective immunoadsorbents based on agarose-polyaldehyde microsphere beads: synthesis and affinity chromatography are disclosed by Margel, et al., Analytical Biochemistry (1983) 128:342-350.
U.S. Patent No. 4,264,766 (Fischer) discloses immunological diagnostic reagents.
U.S. Patent No. 4,801,504 (Burdick, et al.) discusses fluorescent labels having a polysaccharide bound to polymeric particles.
Polysaccharide-modified immunoglobulins having reduced immunogenic potential or improved pharmacokinetics is discussed in European Patent No. 0 315 456 B1.
Wang, et al., describe a facile synthesis of an aldehydic analog of platelet activating factor and its use in the production of specific antibodies in Chemistry and Physics of Lipids (1990) 55:265-273.

### SUMMARY OF THE INVENTION

It is an embodiment of the present invention to provide for a method for introducing an amine-reactive functionality into a dextran. The method comprises reacting the dextran with an alkylating agent having a functionality that reacts with an hydroxyl group of the dextran thereby forming an alkylated dextran wherein the alkylating agent has an olefin group and treating the alkylated dextran to convert the olefin group to an amine-reactive functionality.

### DESCRIPTION OF THE SPECIFIC EMBODIMENTS

The present invention provides a simple, inexpensive method for conjugation of a macromolecule to a polysaccharide for use in an immunoassay. Amine reactive functionalities such as an aldehyde are introduced into the polysaccharide for linking to a macromolecule. One advantage of the present invention is that, unlike prior methods, the number of amine reactive functionalities introduced into the polysaccharide can be controlled.

Before proceeding further with the description of the specific embodiments of the present invention, a number of terms will be defined.

Monosaccharide -- a carbohydrate that cannot be hydrolyzed into simpler compounds such as an aldehyde alcohol or a ketone alcohol, e.g., a hexose or a pentose.

Polysaccharide -- a carbohydrate containing three or more monosaccharide units; the polysaccharide can be hydrolyzed into the simpler monosaccharide units. Examples of polysaccharides by way of illustration and not limitation are dextran, starch, glycogen, polyribose and the like.

Dextran -- a polysaccharide consisting of linear 1-6 linked (98%) glucose units; a polymerized glucose.

Alkyl -- a monovalent branched or unbranched radical derived from an aliphatic hydrocarbon by removal of one H atom; includes both lower alkyl and upper alkyl.

Lower alkyl -- alkyl containing from 1 to 5 carbon atoms such as, e.g., methyl, ethyl, propyl, butyl, isopropyl, isobutyl, pentyl, isopentyl, etc.

Upper alkyl -- alkyl containing more than 6 carbon atoms, usually 6 to 20 carbon atoms, such as, e.g., hexyl, heptyl, octyl, etc.

Alkylidene -- a divalent organic radical derived from an aliphatic hydrocarbon, such as, for example, ethylidene, in which 2 hydrogen atoms are taken from the same carbon atom.

Aryl -- an organic radical derived from an aromatic hydrocarbon by the removal of one atom and containing one or more aromatic rings, usually one to four aromatic rings, such as, e.g., phenyl (from benzene), naphthyl (from naphthalene), etc., e.g., phenyl, naphthyl, phenanthryl.

Aralkyl -- an organic radical having an alkyl group to which is attached an aryl group, e.g., benzyl, phenethyl, 3-phenylpropyl, 1-naphthylethyl, etc.

Alkoxy -- an alkyl radical attached to the remainder of a molecule by an oxygen atom, e.g., methoxy, ethoxy, etc.

Aryloxy- an aryl radical attached to the remainder of a molecule by an oxygen atom, e.g., phenoxy, naphthoxy, etc., e.g., m-methoxyphenyl
Aralkoxy -- an aralkyl radical attached to the remainder of a molecule by an oxygen atom, e.g., benzoxy, 1-naphthylethoxy, etc.

Amine reactive functionality -- a functionality reactive with an amine functionality, usually by virtue of nucleophilicity or basicity of the amine, such as, for example, an aldehyde, an a-keto carboxylic acid and the like.

Alkylating agent having a functionality that reacts with an hydroxyl group -- a compound that has a functionality reactive with an hydroxyl group, usually by virtue of nucleophilicity of the neutral or ionized hydroxyl group, such as, for example, an oxiranyl radical, an alkyl radical comprising a leaving group such as, for example, halide (bromide, chloride, iodide); aryl sulfonates; alkyl sulfonates; aryl sulfates; alkyl sulfates; tosylates; acrylic acid derivatives such as acrylamide; vinyl sulfones; and the like.

Linking group -- a portion of a structure which connects two or more substructures. The linking group may vary from a bond to a chain of from 1 to 30 or more atoms, usually from about 1 to 20 atoms, preferably 1 to 10 atoms, each independently selected from the group normally consisting of carbon, oxygen, sulfur, nitrogen, and phosphorous, usually carbon and oxygen. The number of heteroatoms in the linking group normally ranges from about 0 to 8, usually from about 1 to 6, more preferably 2 to 4. The number of atoms in the chain is determined by counting the number of atoms other than hydrogen or other monovalent atoms along the shortest route between the substructures being connected. The atoms of the linking group may be substituted with atoms other than hydrogen such as carbon, oxygen and so forth in the form, e.g., of alkyl, aryl, aralkyl, hydroxyl, alkoxy, aryloxy, aralkoxy, and the like. As a general rule, the length of a particular linking group can be selected arbitrarily to provide for convenience of synthesis with the proviso that there be minimal interference caused by the linking group with the ability of the present polymers to be attached to a biomolecule.

The linking group may be aliphatic or aromatic. When heteroatoms are present, oxygen will normally be present as oxy or oxo, bonded to carbon, sulfur, nitrogen or phosphorous; sulfur will be present as thioether or thiono; nitrogen will normally be present as nitro, nitroso or amino, normally bonded to carbon, oxygen, sulfur or phosphorous; phosphorous will be bonded to carbon, sulfur, oxygen or nitrogen, usually as phosphonate and phosphate mono- or diester. Functionalities present in the linking group may include esters, thioesters, amides, thioamides, ethers, ureas, thioureas, guanidines, azo groups, thioethers, carboxylate and so forth.

Examples, by way of illustration and not limitation, of various linking groups that find use in the present invention are found in U.S. Patent No. 3,817,837, particularly at column 30, line 69, to column 36, line 10, which disclosure is incorporated herein by reference in its entirety.

Preferred linking groups include alkylene, i.e., (CH₂)ₚ wherein p is an integer in the range of 1 to 10, preferably, 1 to 5, wherein one or more hydrogens, usually one hydrogen, on one or more of the carbons, usually one, of the alkylenes may be substituted with OR⁹ wherein R⁹ is H, alkyl or aryl, e.g., methylene, ethylene, propylene, butylene, pentylene, hexylene, phenylene, p-benzylene and so forth; alkyleneoxyalkylene, i.e., (CH₂)_{q}X(CH₂)ᵣ wherein q and r are the same or different and are integers in the range of 1 to 5, preferably, 1 to 3, and wherein one or more hydrogens, usually one hydrogen, on one or both, usually one, of the alkylenes may be substituted with OR⁹ wherein R⁹ is H, alkyl or aryl, preferably H, e.g., methyleneoxymethylene, methyleneoxyethylene, ethyleneoxyethylene, 1-methyleneoxy-2-hydroxyethylene and so forth and X is independently O or S, preferably O.

Substituted -- means that a hydrogen atom of a molecule has been replaced by another atom, which may be a single atom such as a halogen, etc., or part of a group of atoms forming a functionality as described above. Such substituent may be a group or functionality imparting hydrophilicity or lipophilicity. Hydrophilicity may be achieved by a functional group having one or more atoms such as oxygen, nitrogen, sulfur, phosphorus, and so forth; such groups include sulfonate, sulfate, phosphate, amidine, phosphonate, carboxylate, hydroxyl particularly polyols, amine, ether, amide, and the like. Illustrative functional groups are carboxyalkyl, sulfonoxyalkyl, CONHOCH₂COOH, CO-(glucosamine), sugars, dextran, cyclodextrin, SO₂NHCH₂COOH, SO₃H, CONHCH₂CH₂SO₃H, PO₃H₂, OPO₃H₂, hydroxyl, carboxyl, ketone, and combinations thereof. Lipophilicity may be achieved by a functional group such as carbon atoms substituted with hydrogen or halogen and can include alkyl, alkylidene, aryl and aralkyl. The lipophilic group or functionality will normally have one to six straight or branched chain aliphatic groups of at least 6 carbon atoms, more usually at least 10 carbon atoms, and preferably at least 12 carbon atoms, usually not more than 30 carbon atoms.

In one aspect the present invention pertains to a process for the manufacture of polymers comprising a sequence of repeating monosaccharide units, each of which is independently selected from monosaccharide units of the formula: 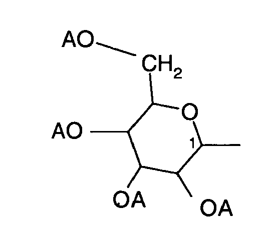 wherein one of the A's is a bond to the C1 glycosidic carbon (as indicated in the above formula) of another of the units and the other A's are independently selected from the group consisting of H and QL, wherein L is a linking group linking O and Q and Q is C(Z)=D wherein D is O or CR¹R² wherein R¹ and R² are independently H, alkyl or aryl and Z is H or C(Y)=O wherein Y is R³, OR³ or NR³R⁴ wherein R³ and R⁴ are independently H, alkyl or aryl and wherein any of L, R¹, R², R³ and R⁴ may be taken together to form a ring with the proviso that at least two of the A's in the polymer are QL. Usually, a ring, when present, is a three to eight member ring, preferably, a five to seven member ring, that can have one or more unsaturations. The ring may be aryl or aralkyl. One or more atoms of the ring may be substituted with a substituent other than hydrogen to form one or more functionalities. The atoms of the ring other than hydrogen are usually carbon but may also include oxygen, sulfur, boron, silicon and/or nitrogen. Exemplary of such rings are benzene, naphthalene, anthracene, phenanthrene, pyridine, pyrimidine furan, indene, indane, pyrrole, thiophene, imidazole, and the like.

The above polymer generally has from about 3 to 50,000, preferably, 25 to 10,000, more preferably, 50 to 1,000 repeating monosaccharide units. Preferably, there are at least two A's in QL in at least one of the monosaccharide units for every 20, more preferably, every 10, monosaccharide units of the polymer.

The following are examples, by way of illustration and not limitation, of polymers in accordance with the present invention.

L is alkylene, e.g., methylene (-CH₂-), ethylene (-CH₂CH₂-), etc.; or alkyleneoxy-hydroxyalkylene, e.g.,1 -methyleneoxy-2-hydroxyethylene (-CH₂-O-CH₂-C(OH)H-), etc.

Q is (i) -CH=O, (ii) -C(O)-C(O)-R³, (iii)-C(O)-C(O)-OR³, (iv) -C(O)-C(O)-NR³R⁴, (v) -CH=CR¹R², (vi) (R¹R²)C=C-C(O)-R³ , (vii) (R¹R²)C=C-C(O)-OR³ , and (viii) (R¹R²)C=C-C(O)-NR³R⁴.

R³ and R⁴ are lower alkyl, preferably methyl.

Particularly preferred polymers in accordance with the present invention are those wherein the polymer is a modified dextran in which the A on the methyleneoxy oxygen of one of the units of the polymer is a bond to theC1 glycosidic carbon of another of the units of the polymer and QL is HC(O)CH₂- or HC(O)CH₂OCH₂C(OH)HCH₂-.

The polymers of the invention can be prepared in a number of ways. The following discussion of one example of a preparation of the above polymers is by way of illustration and not limitation. The method described below is readily carried out and does not compromise the stability of the polysaccharide. The method comprises reacting the polysaccharide with an alkylating agent having a functionality that reacts with an hydroxyl group of the polysaccharide thereby forming an alkylated polysaccharide wherein the alkylating agent has an olefin group and then treating the alkylated polysaccharide to convert the olefin group to an amine-reactive functionality.

The alkylating agent may comprise the structure

L³(Z¹)C=CR⁵R⁶

wherein L³ is a group comprising a functionality reactive with a hydroxyl group of the polysaccharide, R⁵ and R⁶ are independently H, alkyl, aryl or may be taken together with each other or with L³ to form a ring and Z¹ is H or C(W)=O wherein W is R⁷, OR⁷, or NR⁷R^{B} wherein R⁷ and R⁸ are independently H, alkyl or aryl. Usually, a ring, when present, is a three to eight member ring, preferably, a five to seven member ring, that can have one or more unsaturations. The ring may be aryl or aralkyl. One or more atoms of the ring may be substituted with a substituent other than hydrogen to form one or more functionalities. The atoms of the ring other than hydrogen are usually carbon but may also include oxygen, sulfur, boron, silicon and/or nitrogen. Exemplary of such rings are benzene, naphthalene, anthracene, phenanthrene, pyridine, pyrimidine furan, indene, indane, pyrrole, thiophene, imidazole, and the like.

The polymer that is desired will determine the particular alkylating agent to be used. For example, to make the polymer described above wherein QL is HC(O)CH₂-, one utilizes an alkylating agent wherein L³ is methylene having a leaving group such a bromide, Z¹, R⁵ and R⁶ are H. To make the polymer described above wherein QL is HC(O)CH₂OCH₂C(OH)HCH₂-, one utilizes an alkylating agent wherein L³ is CH₂OCH₂(C₂H₃O) wherein (C₂H₃O) is an oxiranyl radical. The latter alkylating agent is preferred when a water soluble polymer is desirable. Maintenance of the water solubility of the dextran can be achieved generally by incorporating a group that imparts hydrophilicity into the dextran during the introduction of the aldehyde group. Accordingly, a preferred alkylating agent is CH₂=CHCH₂OCH₂(C₂H₃O), which results in the introduction of a QL that contains additional oxygen atoms, i.e., HC(O)CH₂OCH₂C(OH)HCH₂-.

For example, dextran is treated with allyl glycidyl ether (CH₂=CHCH₂OCH₂(C₂H₃O) in an acidic aqueous medium. An acid catalyzed opening of the epoxide ring is employed, which results in the addition of a hydroxyl group at one of the carbon atoms of the dextran to one of the two carbon atoms of the epoxide ring to give allyioxydextran. For this step the pH of the medium is about 1 to 4, preferably 1.5 to 2.5. Usually, it is desirable to conduct the reaction in the presence of a Lewis acid such as, for example, Zn(BF₄)₂ , H₂SO₄, SnCl₂, and the like. Accordingly, the pH for the reaction should not be so low or so high as to affect linkages in, or cause other deleterious effects on, the dextran molecule. Where a Lewis acid is employed, the pH should not be so high as to result in a deleterious effect on the Lewis acid such as causing precipitation of unwanted reaction materials. The amount of the Lewis acid employed is about 10-30% (weight/weight) of dextran. Control of the pH permits control of the number of aldehyde groups introduced into the dextran; thus pH control is important.

The time period for the reaction is usually about 6 to 18, preferably, about 10 to 15, hours. It is preferable to conduct the reaction in an atmosphere of inert gas, such as for example, argon, neon, nitrogen and the like. The temperature of the reaction mixture is generally about 70 to 90°C, usually about 75 to 85°C , preferably, about 80°C.

The order of addition for the reagents usually involves dissolving the dextran, Lewis acid and alkylating agent such as allyl glycidyl ether in water with heating at about 60 to 80°C, preferably about 68 to 72°C. After the desired period of time, the reaction mixture is cooled and the resulting allyloxy dextran product is purified, for example, by filtration, ultrafiltration or the like.

The above procedure introduces on the average about one allyloxy group per 5 to 20, usually, 7 to 12, preferably, 8 to 10 polysaccharide units of the dextran molecule. Usually, only one allyloxy group is introduced into a polysaccharide unit that is alkylated, generally, at the hydroxyl group at the 2, 3 or 4 position.

The allyloxy dextran is treated to introduce an aldehyde functionality. To this end the double bond is oxidized by suitable means to cleave the double bond and introduce a terminal aldehyde group. The oxidation can be carried out by any means that produces a terminal aldehyde group but does not affect other parts of the dextran molecule. One convenient way to oxidize the double bond of the allyloxy dextran is to subject the allyloxy dextran to ozonolysis followed by reductive workup such as with dimethyl sulfide. To this end ozone is generated in the reaction mixture at a temperature of about 15 to 30°C, usually about 20 to 28°C , preferably, about 24 to 27°C . For this step the pH of the medium is about 5 to 8, preferably, 6 to 7. The amount of ozone used is generally that amount sufficient to oxidize all of the allyl groups to aldehydes. The time period for the reaction is usually about 5 to 10 hours, preferably, about 5 to 7 hours. It is preferable to conduct the reaction in atmosphere of inert gas. After the desired period of time, the reaction mixture is cooled and ozonide is reduced, for example, by the addition of a reducing agent such as, for example, a sulfide, thiosubstituted phosphine or the like. The resulting dextran aldehyde product is purified by precipitation with an organic solvent, dialysis or the like.

As an alternative to the above method for preparing the present polymers, the above alkylating agent can be replaced by CH(O)CH₂OCH₂(C₂H₃O). The resulting product from this alkylating agent is the desired dextran aldehyde. Accordingly, the desired dextran aldehyde can be obtained by direct alkylation although the aforementioned alkylating agent is preferred because the free aldehyde undergoes side reactions under the acidic conditions.

In an alternate embodiment the amine-reactive functionality introduced is an alpha-keto carboxylic acid functionality. Again, the polymer that is desired will determine the particular alkylating agent to be used. For example, to make the polymer described above wherein QL is HC(O)CH₂OCH₂C(OH)HCH₂-, one can utilize an alkylating agent wherein L³ is CH₂OCH₂(C₂H₃O) wherein (C₂H₃O) is an oxiranyl radical and Z¹ is C(O)OH. For example, dextran is treated with an alkylating agent of the formula CH₂=C(COOH)CH₂OCH₂(C₂H₃O) in an acidic aqueous medium. As described above, an acid catalyzed opening of the epoxide ring is realized, which results in the addition of a hydroxyl group of the dextran to a carbon atom of the epoxide ring to give allyloxydextran. For this step the pH of the medium is about 1 to 4, preferably 1.5 to 2.5. Usually, it is desirable to conduct the reaction in the presence of a Lewis acid under conditions similar to those described above. After the desired period of time, the reaction mixture is cooled and the resulting product is purified by filtration, ultrafiltration or the like. The product is treated to introduce an aldehyde functionality. To this end the double bond is oxidized by suitable means to cleave the double bond and introduce an oxo functionality to yield an alpha keto acid product. The oxidation can be carried out by any means that produces an oxo functionality but does not affect other parts of the molecule. One convenient way to oxidize the double bond of this product to introduce an oxo functionality is to subject the product to ozonolysis as described above. The resulting alpha keto acid product is purified by filtration, ultrafiltration or the like.

As an alternative to the above method for preparing the present polymers, the above mentioned alkylating agent can be replaced by C(O)(COOH)CH₂OCH₂(C₂H₃O). The direct product from this alkylating agent is the alpha keto acid substituted dextran.

In another exemplary approach in accordance with the present invention, a dextran aldehyde polymer can be prepared wherein QL is HC(O)CH₂-. In this approach an alkylating agent containing a leaving group, such as, for example, allyl bromide, is employed. The reaction comprises displacement of bromide of the alkylating agent by an oxygen atom of a polysaccharide unit of the dextran in the presence of a base such as sodium hydroxide, potassium hydroxide, or the like. Dextran is combined with allyl bromide in an aqueous medium in an inert atmosphere in the presence of a sufficient amount of a base to achieve the above mentioned displacement. For this step the pH of the medium is about 10 to 14, preferably 11 to 14. The pH for the reaction should not be so low or so high as to affect linkages in, or cause other deleterious effects on, the dextran molecule. Control of the pH permits control of the number of aldehyde groups introduced into the dextran; thus pH control is important.

The time period for the reaction is usually about 2 to 8 hours, preferably, about 3 to 5 hours. It is preferable to conduct the reaction in an atmosphere of inert gas.

The order of addition for the reagents is not critical. Preferably, the dextran is dissolved in water with heating at about 50 to 80°C, preferably, about 60 to 70°C. Then, the pH of the medium is adjusted to the desired level. Next, the allyl bromide is added to the reaction mixture and the temperature is raised to the desired reaction temperature if necessary. After the desired period of time, the reaction mixture is cooled and the resulting allyloxy dextran product is purified by precipitation with an organic solvent, ultrafiltration or the like.

The above procedure introduces on the average about one allyl group per 3 to 20, usually, 7 to 15, preferably, 8 to 12 polysaccharide units of the dextran molecule.

The allyl dextran is treated to introduce an aldehyde functionality as described above for the allyloxy dextran. The resulting dextran aldehyde product is purified by precipitation with an organic solvent, ultrafiltration or the like, as described previously.

The invention is demonstrated further by the following illustrative example.

### Example

Parts and percentages herein are by weight unless otherwise indicated. Temperatures are in degrees Centigrade (°C).

### Preparation of Protein A - Dextran Conjugate

### Materials and Equipment

Dextran T-500 from Pharmacia, catalog # 17-0320-02
Allyl glycidyl ether from Aldrich, catalog # A3,260-8
Zinc tetrafluoroborate, Zn(BF₄)₂, 40 wt% solution from Aldrich, catalog # 33,386-7
Sodium hydroxide from Mallinckrodt AR (lot 7707 KMRT) was used to make 10M and 1.0M NaOH solution in water
Dimethyl sulfide, (CH₃)₂S, was from Aldrich, catalog # M8,163-2
1-Heptanol was obtained from Aldrich, catalog # H280-5
Water (deionized) was obtained from a Millipore Filtration Unit
UV-Vis Spectra were recorded on a Hewlett Packard 8452A diode array spectrophotometer
Ozonator used was Polyozone Model T-816 Serial 3641 from Polymetrics, Colorado Springs, CO
Minikros Lab System (Microgon Inc. cat. # SYLS 121 01 N) and Minikros tangential flow modules (M25S 300 01 N, M25S 600 01 N , M21 M 300 01 N) were also from Microgon Inc., Laguna Hills, CA
Oxygen (extra dry) used for generating ozone was obtained from Altair Gases and Equipment, San Ramon, CA
Sodium cyanoborohydride, NaCNBH₃ was from Aldrich Chemical Company (cat. # 15,615-9)
Hydroxylamine hydrochloride, NH₂OH·HCl was from Mallinckrodt (AR grade, lot # 5258 KVEN)
Protein A was from Repligen Corporation (Product code 15001-10-00, product lot RC7151) supplied as 50 mg/mL solution in water
Sodium Acetate, NaOAc, was from Mallinckrodt (AR grade, lot #7364 KPNJ)
Acetate buffer, 0.1 M, pH 5.0
¹H-NMR and ¹³C-NMR spectra were run in D₂O (from Aldrich, 99.5%) on Bruker WP-250 MHz Spectrometer. The ¹H chemical shifts were recorded with water as the reference peak at 4.67 ppm. Acetone and Ethanol were used as internal reference in the ¹³C NMR spectrum, the anomeric carbon at 99.1 ppm was subsequently used as the reference peak.
Size exclusion Chromatography was performed on a Pharmacia FPLC system consisting of a LKB controller LCC 500 plus, two Pumps P-500 and an automated motor valve MV-7. The column used was Superose 6 HR 10/30 (Pharmacia catalog # 17-537-01). The recorder used was a Pharmacia LKD Rec-102. The detector was a Waters Associates Differential Refractometer R401.
Lyophilization was performed on a 8L VirTis Benchtop Freeze Dryer (Scientific Products).

### Preparation of Dextran Aldehyde

Dextran (400g, 500 kD) was dissolved in 1.5 L of water (Millipore), in a 4L beaker by heating at 70°C with overhead stirring. The dextran was added to water at 70°C in 30-50 g portions, each portion being added after the first portion had dissolved. The overhead stirrer was set at 300-400 rpm.

The hot dextran solution was filtered through a fritted funnel (coarse) into an erlenmeyer flask and the filtered solution was poured into a 3-necked flask preequilibrated at 70°C. The beaker was rinsed with 50 mL of hot water, which was filtered through the fritted funnel into the erlenmeyer. This filtrate was added to the reaction mixture.

The funnel was removed from the side neck of the flask and a dual entry Claisen distillation adapter was inserted in its place. The overhead stirrer was started and set at 600-700 rpm and the temperature of the dextran solution was allowed to reach 70°C. The temperature of the oil bath was set at 72-75°C. The reaction was conducted under argon. The solution of Zn(BF₄)₂, (400 mL, 25 wt% in H₂O, pH 1.8 ± 0.2) was poured into the flask containing the dextran solution using a funnel on the Claisen adapter. The funnel was removed from the Claisen adapter and replaced with an addition funnel (500 mL capacity) with a pressure equalizing side arm.

Allyl glycidyl ether (500 mL of the 1.5 L to be added) was added into the addition funnel (in 3 x 500 mL portions) at 8-10 mL/min, while the reaction temperature was maintained at 70 ± 2°C. The addition of the allyl glycidyl was continued until all of the 1.5 L were added. Then, the temperature of the reaction mixture was increased to 80°C. The reaction was allowed to proceed for 12-13 h at 80°C under argon, while being continuously stirred (600-700 rpm).

The reaction vessel was removed from the oil bath and the reaction mixture was allowed to cool to 30°C. The cooled reaction mixture was then poured into 6.0 L of water (Nalgene bucket with spigot) and stirred manually. The diluted reaction mixture was purified by ultrafiltration using a Microgon tangential flow diafiltration system. The allyloxy dextran was concentrated to 1.0 - 1.5 L.

An aliquot (50 mL) of the filtrate was removed and freeze dried for analytical purposes. The remaining solution of allyloxy dextran in water was stored at 4°C and used for the next step. The ¹H and ¹³C nuclear magnetic resonance (NMR) spectra of the above allyloxy dextran was consistent with the expected product.

The allyloxy dextran was subjected to ozonolysis in a 4.0 L beaker equipped with an overhead stirrer. The mixture was stirred at 300-350 rpm and allowed to attain room temperature. Ozone was generated by an ozonator and was added by means of a gas bubbler immersed into the solution of allyloxy dextran at a pressure of 9.0 psi and a flow rate of 2.0 LPM (liters per minute). 10 mL of heptanol was added as an antifoamer. The reaction was monitored by 13C-NMR; the disappearance of the olefinic resonances at 118 and 134 ppm was used as an indication for the completion of the reaction. The ozone addition was continued for 5-6 h. The reaction mixture was cooled to around 10°C. To this was added 50 mL of dimethyl sulfide and stirring under an argon atmosphere was continued for 10 h. The reaction mixture was allowed to attain room temperature while being stirred (300-350 rpm) over this time. The resulting dextran aldehyde was purified by Microgon ultrafiltration.

## Claims

1. A method for introducing an amine-reactive functionality into a dextran, said method comprising:
(a) reacting said dextran with an alkylating agent having a functionality that reacts with an hydroxyl group of said dextran thereby forming an alkylated dextran wherein said alkylating agent has an olefin group and
(b) treating said alkylated dextran to convert said olefin group to an amine-reactive functionality.

2. The method of Claim 1 wherein step (b) comprises ozonolysis of said alkylated dextran.

3. The method of Claim 1 wherein said alkylating agent comprises the structure:
L³(Z¹)C=CR⁵R⁶
wherein L³ is a group comprising a functionality reactive with a hydroxyl group of said dextran, R⁵ and R⁶ are independently H, alkyl, aryl or may be taken together with each other or with L³ to form a ring and Z¹ is H or C(W)=O wherein W is R⁷, OR⁷, or NR⁷R⁸ wherein R⁷ and R⁸ are independently H, alkyl or aryl.

4. The method of Claim 3 wherein L³ comprises an epoxide or an alkyl radical comprising a leaving group.

5. The method of Claim 3 wherein L³ is an alkyl radical comprising a leaving group and said leaving group is selected from the group consisting of bromide, chloride, iodide, aryl or alkyl sulfonates and sulfates.

6. The method of Claim 5 wherein L³ is CH₂OCH₂(C₂H₃O) wherein (C₂H₃O) is an oxiranyl radical.

7. The method of Claim 5 wherein L³ is CH₂Br.

8. The method of Claim 1 wherein said amine-reactive functionality is an aldehyde.

9. The method of Claim 8 wherein said olefin is subjected to ozonolysis to yield said aldehyde functionality.

10. The method of Claim 1 wherein said amine-reactive functionality is an alpha-keto carboxylic acid functionality.
